# EUROPEAN PATENT SPECIFICATION

(11) **EP 1 262 775 B1**
(45) Date of publication and mention of the grant of the patent: **09.04.2008**
(21) Application number: 02077121.8
(22) Date of filing: 30.05.2002
(51) Int. Cl.: G01N 33/24, G01N 1/08, E21B 49/02

(54) **Method and device for soil sampling**
Vorrichtung und Verfahren zur Bereitstellung von Bodenproben
Procédé et dispositif de prélèvement d'échantillons de sol

(30) Priority: 01.06.2001 NL 1018200
(43) Date of publication of application: 04.12.2002
(73) Proprietor: STAAT DER NEDERLANDEN te dezen vertegenwoordigd door de Directeur-Generaal van de Rijkswaterstaat, 2517 JR The Hague (NL)
(72) Inventor: Vink, Joseph Pierre Marie, 3815 BJ Amersfoort (NL)
(74) Representative: Brookhuis, Hendrik Jan Arnold

(56) References cited:
- EP-A- 0 278 557
- EP-A- 0 378 747
- DE-A- 10 002 883
- DE-A- 10 048 089
- DE-A- 19 615 061
- US-A- 5 479 814
- US-A- 6 035 950

## Description

The present invention relates to a method for soil sampling, for determining a quantity of environmentally foreign substances, such as, for example, heavy metals, present in a soil.

In order to determine, for example, the degree of pollution of an underwater bed, a soil sample is currently taken by removing a quantity of soil material from the bed. The absolute quantity of various substances present in the soil material is subsequently measured. A degree of pollution of the soil can be determined on the basis of the measured quantities.

However, an absolute quantity of pollution derived from such a measurement tells us little in practice about the effects of that pollution on the specific location at which the soil sample was taken and on the flora and fauna present there, and it is therefore not sufficiently representative of the real situation.

A method of determining the influence of the pollution of the soil on in particular organisms living in the soil is to take a soil sample, after which the soil material obtained is sieved and homogenized. Test organisms are then introduced into the soil material. After a certain time, these organisms are caught, and it is determined to what degree they have absorbed substances such as, for example, heavy metals. This is a measure of the effect of the pollution of the soil. This method is also indicated by the term bioassay. Local conditions prevailing in the soil are no longer present in the soil sample when the soil sample is disturbed and brought into contact with air by, for example, digging, sieving and homogenizing. In the case of this method the results can therefore hardly be described as sufficiently representative of the real situation.

A method in which a soil sample is taken from a soil without disturbing the sample, and in which the quantity of certain substances in the undisturbed soil sample is measured, is known from US-A-6,000,481.

The soil material under real local conditions consists of sediment particles and a certain quantity of interstitial water that is present in the pores between the sediment particles. The total quantity of a substance under those local conditions can be divided into a fraction that is present in the sediment, a fraction that is present in the interstitial water, and an inert fraction. If the risks formed by the various fractions, for example for organisms present in the soil, have to be determined accurately, it is important for the ratios of these fractions relative to each other to be determined in the course of time, and for the total absorption of the substance in test organisms introduced into the sample to be measured after a certain time.

The object of the present invention is to provide a method for analysing soil material, in which various fractions of substances can be measured in an undisturbed and, if desired, unoxidized soil sample.

This object is achieved by the method according to the invention, which comprises at least the following steps:
- inserting into a soil a hollow body with a body wall that delimits a sampling chamber, in order to take a soil sample,
- closing off the end faces of the hollow body by means of a top plate and a bottom plate,
- removing the hollow body from the soil,
- introducing a number of measuring probes into the soil sample through apertures in the body wall,
- connecting extraction elements to the measuring probes,
- extracting a quantity of the environmentally foreign substances from the soil sample at least once through the measuring probes by means of the extraction elements,
- analysing the environmentally foreign substances and determining the time-dependent change in the quantity of environmentally foreign substances in the soil sample.

Different fractions of substances can be measured in the course of time by having the extraction of the quantity of environmentally foreign substances carried out periodically.

By closing off the hollow body by means of the bottom plate and top plate before said hollow body is removed from the soil, it is ensured that the soil sample remains undisturbed and unoxidized. Measuring by means of measuring probes prevents disturbance of the soil sample during the measurements. The fractions of the environmentally foreign substances are measured in the course of time by analysis of the quantity of environmentally foreign substances periodically extracted from the soil sample by means of the extraction elements. In this case the extraction of the environmentally foreign substances from the soil sample can be carried out by extracting environmentally foreign substances from possibly anoxic interstitial water of the soil sample.

Another object of the invention is to measure the quantity of environmentally foreign substances introduced into an undisturbed and, if desired, unoxidized soil sample that have been absorbed by test organisms.

This object is achieved by the method described above, in which, before the start of the periodic extraction of environmentally foreign substances, test organisms are introduced into the sampling chamber through a closable aperture in the top plate, and in which the test organisms are removed from the soil sample after the completion of the periodic extraction of environmentally foreign substances, and the quantity of environmentally foreign substances absorbed in the test organisms is measured.

A device for carrying out the method according to the invention comprises:
- a hollow body with a body wall that delimits a sampling chamber, for accommodating a soil sample, which body wall is provided with several apertures and at the top side and bottom side is provided with a removable top plate and a removable bottom plate respectively,
- measuring means comprising a number of measuring probes and a number of extraction elements, for determining the presence and quantity of certain substances in the sample, at least one extraction element being connectable to each measuring probe,
- a pump for creating a partial vacuum in the measuring probes,
the apertures being suitable for at least one measuring probe to be passed through to the sampling chamber from outside the body wall, and being distributed over the body wall.

In a preferred embodiment the body wall is substantially cylindrical, the apertures being distributed in the tangential direction around the circumference of the body wall and lying at a distance from each other in the longitudinal direction.

This measure ensures that measurements can be made at different depths in the soil sample, the measuring probes inserted through the apertures ending in a centre area in the soil sample around the axis of the body, with the result that measurements with different measuring probes in each case relate to different depths in the same centre area, in which centre area there is the least influence of the body wall on the measurements.

The top plate is preferably provided with a gas feed valve and an excess pressure valve. By means of the gas feed valve, a gas can be fed into the space in the sampling chamber above the soil sample, for example in order to create an anoxic situation. The gas pressure above the soil sample is kept constant by means of the excess pressure valve.

The device makes it possible to carry out measurements on the soil sample on location immediately after the sample has been taken, after which the soil sample is removed again from the sampling chamber and only the extraction elements are retained for subsequent analysis in a laboratory. This therefore means that a single (absolute) quantity of certain substances, such as, for example, heavy metals, is measured at a certain time. It is advantageous here that it is not the soil sample, which is difficult to preserve, in particular the interstitial water between the sediment particles contained in it, that has to be retained, but only the stable extraction elements, which are then analysed.

It is also possible to transport the device containing a soil sample to a laboratory and there to measure processes in the sample that change with time by periodically connecting extraction elements to the measuring probes.

The invention will be explained in greater detail in the description below with reference to the drawing, in which:
Fig. 1 shows a view in cross section of a sampling device for carrying out the invention;
Fig. 2 shows a top view of a sampling device according to Fig. 1;
Fig. 3 shows a cross section of a top plate in accordance with the line A-A in Fig. 2;
Fig. 4a shows a top view of a clamping piece;
Fig. 4b shows a view in cross section of a clamping piece of Fig. 4a;
Fig. 5a shows an opened-out view of the body wall with feed-through apertures;
Fig. 5b shows an opened-out view of a different embodiment of the body wall with feed-through apertures;
Fig. 6 shows a side view of a feed-through aperture for electrodes in the top plate of the sampling device;
Fig. 7 shows a longitudinal section of a measuring probe.

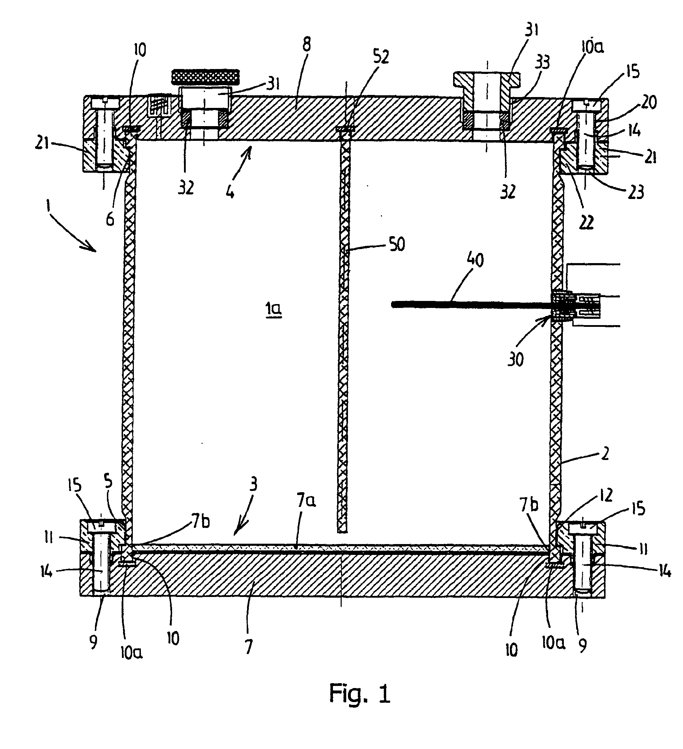

Figures 1 to 3 show a thin-walled hollow body 1 with a body wall 2. The body wall 2 encloses a sampling chamber 1a. In the exemplary embodiment shown, the hollow body 1 is cylindrical, but it can also be another shape. On the end faces of the body 1, the body wall 2 is provided on the outside circumference with an annular recess 5 near a bottom end 3, and is provided with an annular recess 6 near a top end 4.

The bottom end 3 of the hollow body 1 can be closed off by means of a sealing plate 7a and a bottom plate 7. At the circumferential edge 7b, the sealing plate 7a presses against the body wall 2. The sealing plate 7a is made of, for example, polyethylene. Threaded holes 9, which extend through the entire thickness of the bottom plate 7, are fitted around the circumference of the bottom plate 7. The bottom plate 7 is also provided with a circular groove 10, which is suitable for accommodating the end 3 of the body wall 2 in a fitting manner. A sealing ring 10a is accommodated in the groove 10, in order to guarantee a gastight and liquid-tight seal.

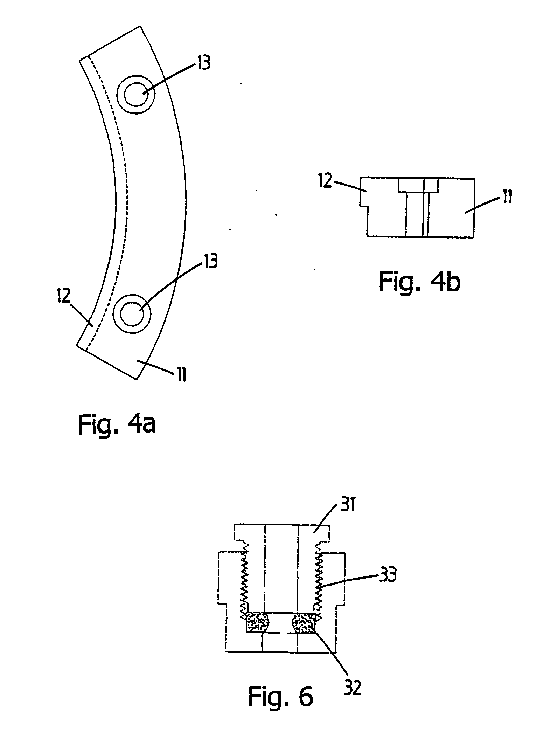

The bottom plate 7 is fixed on the body 1 by means of clamping pieces 11. The clamping pieces 11 are in the form of ring segments, as shown in Figs. 4a and 4b. The clamping pieces 11 are provided with a projection 12 that fits into the annular recess 5. In addition, clamping pieces 11 are provided with holes 13, which extend through the clamping pieces 11, in order to pass through screws 14. The holes 13 have a larger diameter on one side, so that the head 15 of each screw 14 can be countersunk in them. The clamping pieces 11 and the bottom plate 7 are connected to each other by tightening the screws 14. During the fitting of the bottom plate 7 on the body 1, the bottom plate 7 is placed against the body 1, so that the end is accommodated in the circular groove 10, as shown in Fig. 1. At the same time, the clamping pieces 11 with their projection 12 are snapped into the annular recess 5, after which the clamping pieces 11 and the bottom plate 7 are clamped against each other by means of screws 7, so that the bottom plate 7 is clamped down against the body 1.

The top end of the body can be closed off by a top plate 8. Holes 20 are made around the circumference in the top plate 8, which holes extend through the entire thickness of the top plate 8. The holes 20 have a larger diameter on one side, so that the head 15 of a screw 14 can be countersunk in them. In addition, the top plate 8 is provided with a circular groove 10, which is suitable for accommodating the end of the body wall 2 in a fitting manner.

The top plate 8, just like the bottom plate 7, is fixed on the body 1 by means of clamping pieces 21. The clamping pieces 21 are in the form of ring segments. The clamping pieces 21 are provided with a projection 22 that fits into the annular recess 6. In addition, the clamping pieces 21 are provided with holes 23, which extend through the thickness of the clamping pieces 21 and are provided with screw thread. The top plate 8 can be snapped onto the body 1 by means of the clamping pieces 21 in the same way as the bottom plate 7, and then clamped down by means of screws.

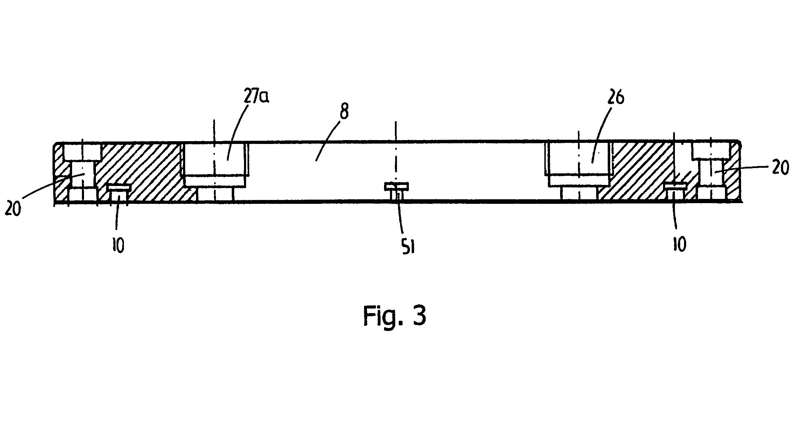

It can be seen in Fig. 1 that a partition 50 is provided in the sampling chamber 1a. Said partition 50 may, if desired, be placed when the top plate 8 is being fitted in the sampling chamber 1a. On the side facing the sampling chamber 1a, the top plate 8 is provided with a groove 51 for accommodating the top edge of the partition 50, as Fig. 3 also shows. The groove is provided with a sealing strip 52 (see Fig. 1), so that the connection between the top plate 8 and the partition 50 is gastight. The sampling chamber 1a can be divided into two compartments by means of the partition 50, so that, for example, comparative measurements can be carried out on a soil sample.

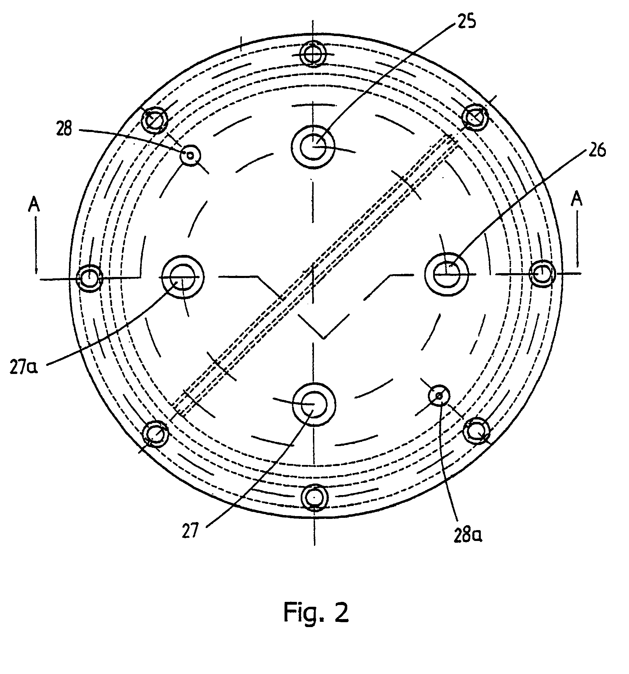

It can be seen in Fig. 2 that the top plate 8 is provided with a connection aperture 28, to which a gas feed valve (not shown) can be connected. In addition, the top plate 8 is provided with a second connection aperture 28a, to which an excess pressure valve (not shown) can be connected. Fig. 2 shows the state of the device after the top and bottom side have been closed off after the accommodation of a soil sample in the sampling chamber. In addition, the top plate 8 is provided with four identical feed-through structures 25, 26, 27, 27a for attaching measuring electrodes as shown in Figs. 1 and 6. The feed-through structure comprises a hollow screw 31, a bore 33 in the top plate 8, which is threaded on the inside, and a sealing ring 32. The seal between the feed-through structure and an electrode is made gastight and liquid-tight by exerting pressure with the hollow screw 31 on the sealing ring 32 (see 32, Fig. 6). This has the advantage that a good seal is obtained, irrespective of the diameter of the electrode used.

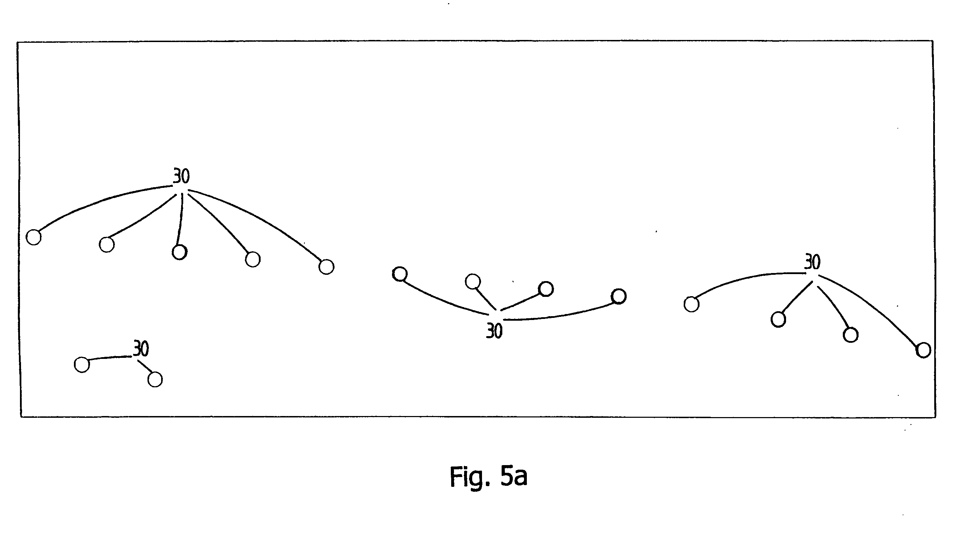

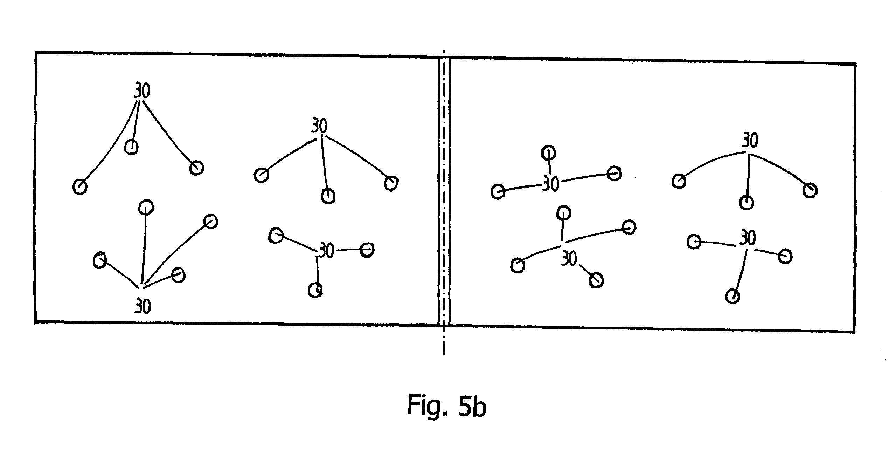

As Figs. 1, 5a and 5b show, several apertures 30 are provided in the body wall 2, which apertures serve as the feed-through for measuring probes 40. The apertures 30 are tangentially distributed around the circumference of the body wall 2 and are at a distance from each other vertically, as is clear from Fig. 5a, in which the opened-out state of the body wall 2 is shown in a first embodiment. The apertures 30 therefore form a spiral around the circumference in the embodiment shown in Fig. 5a. During the taking of a soil sample, these apertures 30 are closed off in such a way by closure caps (not shown in any further detail) that the inside of the body wall 2 is entirely smooth. Fig. 5b shows a body wall 2 with an arrangement of the apertures 30 that can be used if the sampling chamber is divided into two compartments by the partition 50. In this case the apertures 30 are positioned relative to each other in such a way that the measuring probes have the same position in the two compartments. This makes it possible to carry out comparative measurements on the two compartments of the sampling chamber 1a, and thus on two parts of the same soil sample.

A soil sample is taken by inserting the body 1, without the sealing plate 7a, bottom plate 7 and top plate 8, the apertures 30 of which have been closed off, so far into the soil that the sampling chamber 1a is partially filled with soil material. Surface water and air are still present above the soil material. The body 2 is then closed off on the underside by the sealing plate 7a and pulled out of the soil. The top plate 8 and bottom plate 7 are then snapped, with the clamping pieces 11 and 21 respectively, onto the body and subsequently clamped down on the body 2 by tightening the screws 14.

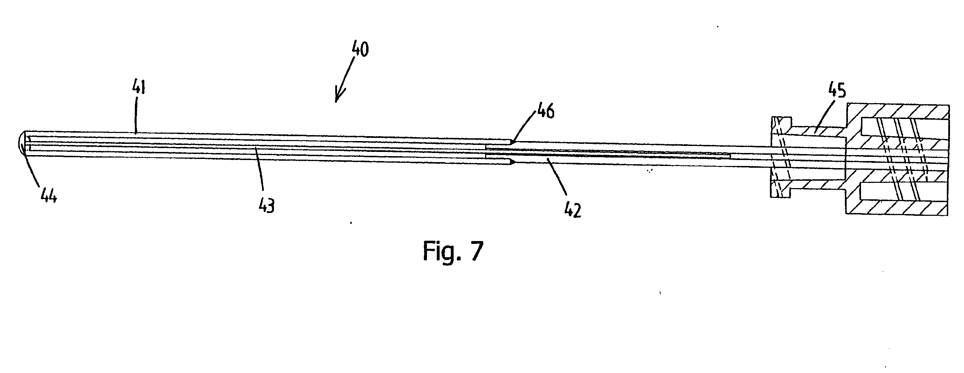

In order subsequently to carry out measurements, the measuring probes are introduced substantially horizontally into the sample through the feed-through apertures 30 in the body wall 2. Fig. 7 shows a measuring probe 40. The measuring probes are hollow, bar-shaped elements with a wall 41, made of a semi-permeable polymer, fixed by means of an adhesive connection 46 on an impermeable connecting piece 42, and having an internal reinforcement 43 of, for example, Teflon or glass fibre. The end of the measuring probe 40 that projects into the sample is closed off by a resin droplet 44. The measuring probes 40 are connected to a vacuum pump, for the purpose of creating a partial vacuum in the measuring probes 40 in relation to the pressure in the soil sample, so that interstitial water from the soil sample with substances dissolved in said interstitial water is sucked in through the semi-permeable wall. The space in the sampling chamber 1a between the cover plate 8 and the soil sample is filled as required with gas, such as, for example, nitrogen gas for the purpose of creating, for example, anoxic conditions, but oxygen can also be fed in, in order to obtain different conditions in the sample. This gas is supplied through a gas feed valve that is connected to the connection aperture 28. The pressure above the sample is kept constant by an excess pressure valve that is connected to the second connection aperture 28a.

Extraction elements are connected to the measuring probes 40, by means of which the initial state in the sample is established. The measuring probe 40 has a connecting piece 45 (see Fig. 7) to which the extraction element can be connected. The extraction elements are, for example, tubular elements made of acrylate and having at one end a connection for connecting to the connecting piece 45 of the measuring probe 40, and having at the other end a connection for connecting to a hose. A sample bottle can be connected to the hose for the collection of residual liquid from the interstitial water that has run through the extraction element. The extraction elements comprise exchange material that provides for the physical separation of different types of ions in the interstitial water. The exchange material and the residual liquid can be analysed by determining the environmentally foreign substances present in the interstitial water.

Test organisms can then be introduced into the sampling chamber 1a through the gastight access apertures 25, 26, 27, 27a. After some time has elapsed, a new set of extraction elements can be connected to the measuring probes 40, by means of which a second state is established in the sample. The connection of the extraction elements is repeated periodically, so that the state can be established periodically and subsequently a situation changing in time in the sample, in other words the time-dependent change of the fractions present in the sample, can be determined by analysis of the contents of the extraction elements.

Thereafter, the organisms can be removed from the soil sample and can be subjected to a so-called destructive analysis, in which the absorbed quantity of, for example, heavy metals or other pollutants in the organisms is determined.

It is clear from the above that following the chemical or biochemical composition of the soil sample in the course of time and carrying out a bioassay can be performed simultaneously. Only in this way is it possible to deduce a causal connection between the pollution present in the soil and the toxicological effect of that pollution on the organisms present in the soil.

## Claims

1. Method for soil sampling, in order to determine the quantity of environmentally foreign substances, such as, for example, heavy metals present in a soil, the method comprising at least the following steps:
- inserting into a soil a hollow body (1) with a body wall (2) that delimits a sampling chamber (1a), in order to take a soil sample,
- closing off the end faces of the hollow body (1) by means of a top plate (8) and a bottom plate (7),
- removing the hollow body (1) from the soil,
- introducing a number of measuring probes (40) into the soil sample through apertures (30) in the body wall (2),
- connecting extraction elements to the measuring probes (40),
- extracting a quantity of the environmentally foreign substances from the soil sample at least once through the measuring probes (40) by means of the extraction elements,
- analysing the environmentally foreign substances and determining the time-dependent change in the quantity of environmentally foreign substances in the soil sample.

2. Method according to claim 1, in which the extraction of the quantity of environmentally foreign substances from the soil sample is carried out periodically.

3. Method according to claim 1 or 2, in which, after the measuring probes (40) have been placed in the soil sample, a gas is fed into the sampling chamber (1a) through a gas feed valve in the top plate (8).

4. Method according to claim 2 or 3, in which, before the start of the periodic extraction of the quantity of environmentally foreign substances, test organisms are introduced into the sampling chamber (1a) through a closable aperture (25, 26, 27, 27a) in the top plate (8).

5. Method according to claim 4, in which the test organisms are removed from the soil sample after the completion of the periodic extraction of environmentally foreign substances, and the environmentally foreign substances absorbed in the test organisms are measured.

6. Method according to claim 1, in which the extraction of a quantity of environmentally foreign substances from the soil sample by means of the extraction elements is carried out directly after the taking of the soil sample on location, and the analysis of the environmentally foreign substances in the soil sample is carried out with the aid of the extract at another location, for example in a laboratory.

7. Sampling device for carrying out a method according to claim 1, comprising:
- a hollow body (1) with a body wall (2) that delimits a sampling chamber (1a), for accommodating a soil sample, which body wall (2) is provided with several apertures (30) and at the top side and bottom side is provided with a removable top plate (8) and a removable bottom plate (7) respectively,
- measuring means comprising a number of measuring probes (40) and a number of extraction elements, for determining the presence and quantity of certain substances in the sample, at least one extraction element being connectable to each measuring probe (40),
- a pump for creating a partial vacuum in the measuring probes (40),
the apertures (30) being suitable for at least one measuring probe (40) to be passed through to the sampling chamber (1a) from outside the body wall (2), and being distributed over the body wall (2).

8. Device according to claim 7, in which the body wall (2) is substantially cylindrical, and the apertures (30) are distributed in the tangential direction around the circumference of the body wall (2), and lie at a distance from each other in the longitudinal direction.

9. Device according to claim 7. or 8, in which the top plate (8) is provided with a gas feed valve and an excess pressure valve.

10. Device according to one or more of claims 7 - 9, in which a partition (50) is provided in the sampling chamber (1a), in order to divide the sampling chamber (1a) into compartments.

## Patentansprüche

1. Verfahren zur Bodenprobenentnahme, um die Menge umweltfremder Substanzen zu bestimmen, wie z.B. in einem Boden vorhandener Schwermetalle, die Methode umfassend wenigstens die folgenden Schritte:
- einen Hohlkörper (1) mit einer Körperwandung (2), die eine Probenkammer (1a) begrenzt, in einen Boden einführen, um eine Bodenprobe zu entnehmen
- die Endflächen des Hohlkörpers (1) durch eine Deckelplatte (8) und eine Bodenplatte (7) verschließen,
- den Hohlkörper (1) aus dem Boden herausnehmen,
- eine Anzahl von Meßsonden (40) durch Öffnungen (30) in der Körperwandung (2) in die Bodenprobe einbringen,
- Extraktionselemente mit den Meßsonden (40) verbinden,
- wenigstens einmal eine Menge der umweltfremden Substanzen aus der Bodenprobe durch die Meßsonden (40) mittels der Extraktionselemente extrahieren,
- die umweltfremden Substanzen analysieren und die zeitabhängige Änderung der Menge der umweltfremden Substanzen in der Bodenprobe bestimmen.

2. Verfahren gemäß Anspruch 1, bei welchem die Extraktion der Menge umweltfremder Substanzen aus der Bodenprobe periodisch durchgeführt wird.

3. Verfahren gemäß Anspruch 1 oder 2, bei welchem, nachdem die Meßsonden (40) in der Bodenprobe plaziert worden sind, ein Gas durch ein Gaszuführventil in der Deckelplatte (8) in die Probenkammer (1a) eingeleitet wird.

4. Verfahren gemäß Anspruch 2 oder 3, bei welchem vor dem Beginn der periodischen Extraktion der Menge umweltfremder Substanzen durch eine schließbare Öffnung (25, 26, 27, 27a) in der Deckelplatte (8) Testorganismen in die Probenkammer (1a) eingebracht werden.

5. Verfahren gemäß Anspruch 4, bei welchem nach Beendigung der periodischen Extraktion umweltfremder Substanzen die Testorganismen aus der Bodenprobe entfernt werden, und die umweltfremden Substanzen, die von den Testorganismen absorbiert wurden, gemessen werden.

6. Verfahren gemäß Anspruch 1, bei welchem die Extraktion einer Menge umweltfremder Substanzen aus der Bodenprobe mittels der Extraktionselemente unmittelbar nach der Entnahme der Bodenprobe vor Ort ausgeführt wird, und die Analyse der umweltfremden Substanzen in der Bodenprobe mit Hilfe des Extrakts an einem anderen Ort, z.B. in einem Labor, durchgeführt wird.

7. Probenentnahmevorrichtung zur Durchführung eines Verfahrens gemäß Anspruch 1, umfassend:
- einen Hohlkörper (1) mit einer Körperwandung (2), die eine Probenkammer (1a) begrenzt, zum Aufnehmen einer Bodenprobe, welche Körperwandung (2) mit mehreren Öffnungen (30) versehen ist und auf der Oberseite und der Unterseite mit einer entfernbaren Deckelplatte (8) bzw. einer entfernbaren Bodenplatte (7) versehen ist,
- Mittel zum Messen umfassend eine Anzahl von Meßsonden (40) und eine Anzahl von Extraktionselementen, zum Bestimmten des Vorhandenseins und der Menge bestimmter Substanzen in der Probe, wobei wenigstens ein Extraktions-element mit jeder Meßsonde (40) verbindbar ist,
- eine Pumpe zum Erzeugen eines teilweisen Vakuums in den Meßsonden (40), wobei die Öffnungen (30) dafür geeignet sind, daß wenigstens eine Meßsonde (40) von außerhalb der Körperwandung (2) in die Probenkammer (1a) hindurch geführt werden kann, und über die Körperwandung (2) verteilt sind.

8. Vorrichtung gemäß Anspruch 7, bei welcher die Körperwandung (2) im wesentlichen zylindrisch ist, und die Öffnungen (30) in tangentialer Richtung über die Peripherie der Körperwandung (2) verteilt sind, und in longitudinaler Richtung in einem Abstand voneinander liegen.

9. Vorrichtung gemäß Anspruch 7 oder 8, in welcher die Deckelplatte (8) mit einem Gaszuführventil und einem Überdruckventil ausgestattet ist.

10. Vorrichtung gemäß einem oder mehrerer der Ansprüche 7 bis 9, bei welcher eine Trennwand (50) in der Probenkammer (1a) bereitgestellt ist, um die Probenkammer (1a) in Abteilungen zu unterteilen.

## Revendications

1. Procédé pour prélever des échantillons de sol, afin de déterminer la quantité de substances étrangères à l'environnement, telles que, par exemple, des métaux lourds présents dans un sol, le procédé comprenant au moins les étapes suivantes :
- insérer dans un sol un corps creux (1) ayant une paroi (2) qui délimite une chambre de prélèvement d'échantillon (1a), afin de prélever un échantillon de sol,
- fermer les faces d'extrémité du corps creux (1) au moyen d'une plaque supérieure (8) et d'une plaque inférieure (7),
- enlever le corps creux (1) du sol,
- introduire un certain nombre de sondes de mesure (40) dans l'échantillon de sol à travers des ouvertures (30) dans la paroi de corps (2),
- connecter les éléments d'extraction aux sondes de mesure (40),
- extraire une quantité des substances étrangères à l'environnement de l'échantillon de sol au moins une fois à travers les sondes de mesure (40) au moyen des éléments d'extraction,
- analyser les substances étrangères à l'environnement et déterminer le changement en fonction du temps de la quantité de substances étrangères à l'environnement dans l'échantillon de sol.

2. Procédé selon la revendication 1, dans lequel l'extraction de la quantité de substances étrangères à l'environnement de l'échantillon de sol est réalisée périodiquement.

3. Procédé selon la revendication 1 ou 2, dans lequel, après que les sondes de mesure (40) ont été placées dans l'échantillon de sol, un gaz est délivré dans la chambre de prélèvement d'échantillon (1a) à travers une soupape d'alimentation en gaz dans la plaque supérieure (8).

4. Procédé selon la revendication 2 ou 3, dans lequel, avant le début de l'extraction périodique de la quantité de substances étrangères à l'environnement, des organismes d'essai sont introduits dans la chambre de prélèvement d'échantillon (1a) à travers une ouverture pouvant être fermée (25, 26, 27, 27a) dans la plaque supérieure (8).

5. Procédé selon la revendication 4, dans lequel les organismes d'essai sont enlevés de l'échantillon de sol après l'achèvement de l'extraction périodique de substances étrangères à l'environnement, et les substances étrangères à l'environnement absorbées dans les organismes d'essai sont mesurées.

6. Procédé selon la revendication 1, dans lequel l'extraction d'une quantité de substances étrangères à l'environnement de l'échantillon de sol au moyen des éléments d'extraction est réalisée directement après le prélèvement de l'échantillon de sol dans un emplacement, et l'analyse des substances étrangères à l'environnement dans l'échantillon de sol est réalisée à l'aide de l'extrait au niveau d'un autre emplacement, par exemple dans un laboratoire.

7. Dispositif de prélèvement d'échantillon pour réaliser un procédé selon la revendication 1, comprenant :
- un corps creux (1) ayant une paroi (2) qui délimite une chambre de prélèvement d'échantillon (1a), pour loger un échantillon de sol, laquelle paroi de corps (2) est dotée de plusieurs ouvertures (30) et, sur le côté supérieur et le côté inférieur, est dotée d'une plaque supérieure détachable (8) et d'une plaque inférieure détachable (7) respectivement,
- un moyen de mesure comprenant un certain nombre de sondes de mesure (40) et un certain nombre d'éléments d'extraction, pour déterminer la présence et la quantité de certaines substances dans l'échantillon, au moins un élément d'extraction pouvant être lié à chaque sonde de mesure (40),
- une pompe pour créer un vide partiel dans les sondes de mesure (40),
les ouvertures (30) étant adaptées à au moins une sonde de mesure (40) à faire passer à travers la chambre de prélèvement d'échantillon (1a) depuis l'extérieur de la paroi de corps (2), et étant distribuées sur la paroi de corps (2).

8. Dispositif selon la revendication 7, dans lequel la paroi de corps (2) est sensiblement cylindrique, et les ouvertures (30) sont distribuées dans la direction tangentielle autour de la circonférence de la paroi de corps (2), et se trouvent à une certaine distance les unes des autres dans la direction longitudinale.

9. Dispositif selon la revendication 7 ou 8, dans lequel la plaque supérieure (8) est dotée d'une soupape d'alimentation en gaz et d'une soupape d'excès de pression.

10. Dispositif selon une ou plusieurs des revendications 7 à 9, dans lequel une cloison (50) est disposée dans la chambre de prélèvement d'échantillon (1a), afin de diviser la chambre de prélèvement d'échantillon (1a) en compartiments.
